# EUROPEAN PATENT SPECIFICATION

(11) **EP 4 198 016 B1**
(45) Date of publication and mention of the grant of the patent: **04.06.2025**
(21) Application number: 22208962.5
(22) Date of filing: 22.11.2022
(51) Int. Cl.: C07C 211/63, C07C 303/40, C07C 311/08

(54) **NIMESULIDE SALTS AND METHOD OF OBTAINING NIMESULIDE SALT CRYSTALS**
NIMESULIDSALZE UND VERFAHREN ZUR HERSTELLUNG VON NIMESULIDSALZKRISTALLEN
SELS DE NIMESULIDE ET PROCÉDÉ D'OBTENTION DE CRISTAUX DE SEL DE NIMESULIDE

(30) Priority: 16.12.2021 PL 43987021
(43) Date of publication of application: 21.06.2023
(73) Proprietor: Uniwersytet Gdanski, 80-309 Gdansk (PL)
(72) Inventor: Sikorski, Artur, 83-100 Juszkowo-Pruszcz Gdanski (PL); Rybczynska, Malgorzata, 82-100 Malbork (PL)
(74) Representative: Czarnik, Maciej

(56) References cited:
- EP-A1- 0 869 117
- EP-A1- 0 937 709

## Description

The object of the invention is new substances: new nimesulide salts and a method of obtaining them in the form of crystals.

Nimesulide - systematic name: (2-phenoxy-4-nitro)methanesulfoanilide - is a drug belonging to the group of non-steroidal anti-inflammatory drugs (NSAIDs), available in pharmacies by prescription. It has antipyretic, analgesic and anti-inflammatory properties, as described in Kress, H. G.; Baltov, A.; Basinski, A.; Berghea, F.; Castellsague, J.; Codreanu, C.; Copaciu, E.; Giamberardino, M. A.; Hakl, M.; Hrazdira, L.; Kokavec, M.; Lejčko, J.; Nachtnebl, L.; Stančík, R.; Švec, A.; Tóth, T.; Vlaskovska, M. V.; Woroń, J. (2016) Acute pain: a multifaceted challenge - the role of nimesulide, Current Medical Research and Opinion, 32(1), 23-36.

Like most NSAIDs, the mechanism of action of nimesulide is based on inhibition of the activity of cyclooxygenase, an enzyme involved in the synthesis of prostaglandins from cell membrane lipids. There are two isoforms of this enzyme COX-1 and COX-2. The first enzyme is active under physiological conditions in many tissues. COX-2 is an enzyme whose activity increases rapidly in inflamed tissues. Nimesulide, unlike other NSAIDs, acts preferentially towards COX-2 than COX-1, causing inhibition of prostaglandin synthesis from inflamed tissues, as described in Vane, J. R.; Botting R. M. (1998) Mechanism of Action of Nonsteroidal Anti-Inflammatory Drugs, The American Journal of Medicine, 104, 2-8.

The drug is used orally in the form of granules or tablets, usually at a dose of 200 mg per day. The drug, in gel form for the topical treatment of pain and swelling, is used in a dose of 3 g of gel, 2-3 times daily. The substance is mainly used for the symptomatic treatment of pain for up to 15 days. Nimesulide is a substance classified as a weak acid and exhibiting poor water solubility of 0.01 mg/mL, as shown in Purcaru, S. O.; Ionescu, M.; Raneti, C.; Anuta, V.; Mircioiu, I.; Belu, I. (2010) Study of nimesulide release from solid pharmaceutical formulations in tween 80 solutions, Current Health Sciences Journal, 36(1), 42-9.

Tetramethylammonium ion is a cholinomimetic. This group of drugs mimics the action of acetylcholine. The drug causes stimulation and subsequent blockade of nicotinic and muscarinic acetylcholine neurotransport receptors, as described in Anthoni, U.; Bohlin, L.; Larsen, C.; Nielsen, P.; Nielsen, N. H.; Christophersen C. (1989) Tetramine: Occurrence in marine organisms and pharmacology, Toxicon, 27(7), 707-716.

Tetraethylammonium ion shows interesting therapeutic properties - it was the first drug used as a ganglionic blocker as described in D'Arcy, P. F.; Taylor, E. P. (1962) Quaternary ammonium compounds in medicinal chemistry, Journal of Pharmacy and Pharmacology, 14, 129-146. The compound is an inhibitor of nicotinic acetylcholine receptors (Akk, G.; Steinbach, J. H. (2003) Activation and block of mouse muscle-type nicotinic receptors by tetraethylammonium, The Journal of Physiology, 551, 155-168).

The main obstacle to the use of nimesulide is its poor water solubility and poor solubility in solvents/raw materials typically used in pharmaceutical preparations.

From publication EP 0869117 B1 nimesulide choline salt, a process for the preparation thereof and pharmaceutical compositions containing it are described Nimesulide choline salt has physicochemical and pharmacological properties, such as high water-solubility, up to 50% w/v, at room temperature and at a physiologically compatible pH, high stability of the aqueous solutions and high bioavailability.

Nimesulide preparations are also known to be dispersions of active ingredient particles in a component which, in the case of creams, contains a hydrophilic polymer, an oil substance, a surfactant, an alkali substance and water.

Patent description PL 190603 describes topical preparations of nimesulide in the form of gel systems containing a carboxyvinyl polymer neutralised with an aqueous solution of weak bases, a solvent selected from the group consisting of ethanol, isopropanol, diethylene glycol monoethyl ether, and possibly containing caprylic/capric esters and/or glyceryl(8)OE, stabilising agents and/or preservatives, and having a water content in the range of 40 to 95% by weight. A method of preparation comprising the steps is also described:
- formation of a dispersed aqueous phase containing a carboxyvinyl polymer as a gel-forming agent;
- addition of an alcoholic solvent, selected from the group consisting of ethanol and isopropanol, dispersion of the nimesulide active ingredient, possibly addition of preservatives and stabilising agents;
- neutralisation of the resin with an aqueous solution of a weak base.

From patent description PL 202852 is known a composition for topical application which comprises nimesulide in an amount of 0.1 to 5% by weight per composition, glyceryl monooleate in an amount of 16,5 to 59% by weight per weight of the composition and a non-aqueous solvent in an amount of 25 to 82% by weight per weight of the composition selected from the group consisting of C1-C6-alcohol, N-methylpyrrolidone, glycol or glycol ether, C8-C22 glyceride or ethoxylated glyceride.

Research to increase the bioavailability of poorly water-soluble drugs is an important aspect in the design of products with therapeutic properties. One method of achieving this is to complex the pharmaceutically active substance with excipients. The creation of new drug forms - with improved properties and, in particular, with the least possible side effects associated with their intake - is possible by designing multicomponent crystals in the form of salts, solvates, polymorphs, or cocrystals (Thakuria, R.; Sarma, B. (2018). Drug-Drug and Drug-Nutraceutical Cocrystal/Salt as Alternative Medicine for Com-bination Therapy: A Crystal Engineering Approach, Crystals, 8(2), 101, 1-39). Obtaining multicomponent crystals in which at least one of the components is a pharmaceutically active ingredient makes it possible to improve the pharmacodynamic properties of drugs that have a limited therapeutic range and require constant control of blood concentrations, as well as to minimise the side effects of their use (Blagden, N.; de Matas, M.; Gavan, P. T.; York, P. (2007) Crystal engineering of active pharmaceutical ingredients to improve solubility and dissolution rates, Advanced Drug Delivery Reviews, 59(7), 617-630).

From the state of the art described in The Cambridge Structural Database (CSD) to date, it appears that few multicomponent crystals involving nimesulide have been known to date. The CSD is a worldwide database containing a collection of all crystal structures of organic and organometallic compounds. Currently, 14 complexes involving nimesulide can be found in the database: four are structures of two polymorphic varieties of nimesulide studied at room temperature and low temperature, one of a nimesulide derivative methylated on the nitrogen atom, and the remaining five, structures of nimesulide complexes with silver as described in: Dupont, L.; Pirotte, B.; Masereel, B.; Delarger, J.; Geczy, J. (1995) Acta Crystallographica, C51, 507-509; Sanphui, P.; Sarma, B.; Nangia, A. (2011) Phase transformation in conformational polymorphs of nimesulide, Journal of phramace, Journal of Pharmaceutical Sciences, 100(6), 2287-2299; Michaux, C.; Charlier, C.; Julemont, F.; Norberg, B.; Dogne, J. M.; Durant, F. (2001) Acta Crystallographica, E57, 1012-1013 Investigations into the activity of certain complexes demonstrate that they possess not only analgesic but also antitumour properties. Nimesulide-silver complexes described in Banti, C. N.; Papatriantafyllopoulou, C.; Manoli, M.; Tasiopoulos, A. J.; Hadjikakou, S. K. (2016) Nimesulide Silver Metallodrugs, Containing the Mitochondriotropic, Triaryl Derivatives of Pnictogen; Anticancer Activity against Human Breast Cancer Cells, Inorganic Chemistry, 55, 17, 8681-8696 show protective properties against breast cancer development. In addition, 4 new cocrystal structures of nimesulide and pyridine derivatives have been reported in 2020 base as described in: Wang, M.; Ma, Y.; Shi, P.; Du, S.; Wu, S.; Gong J. (2021) Similar but Not the Same: Difference in the Ability to Form Cocrystals between Nimesulide and the Pyridine Analogues, Crystal Growth & Design, 21(1), 287-296.

In none of the above structures is nimesulide present in the ionised form (nimesulide anion).

The essence of the solution according to the invention are new compounds potentially pharmaceutically active from the NSAID group - crystals of tetramethylammonium salt of nimesulide - shown as formula 1 and tetraethylammonium salt of nimesulide - formula 2, in which nimesulide is present in an ionised form, which links the two inventions.

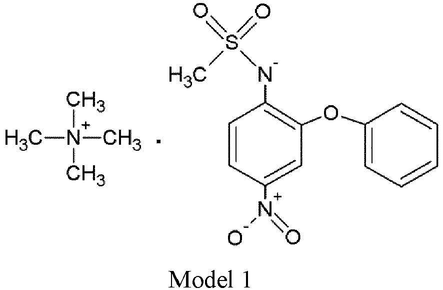

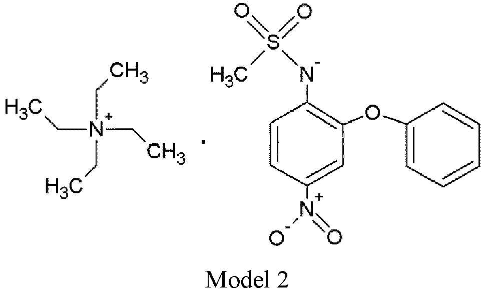

The invention may have applications in the pharmaceutical industry as an analgesic, anti-inflammatory and antipyretic, cholinomimetic, in neurodegenerative and other diseases, as well as in the preparation of drugs with such effects, as confirmed by studies on the properties of the individual components of both substances described in the scientific literature.

Also of the invention is a method for producing a tetramethylammonium salt of nimesulide and a tetraethylammonium salt of nimesulide.

The method for producing crystals of tetramethylammonium salt of nimesulide according to the invention consists in mixing nimesulide and tetramethylammonium hydroxide pentahydrate with each other in any weight %/molar ratio depending on the number of grams of product to be obtained and the yield of the reaction, and then dissolving this mixture in any solvent or mixture of solvents in such a volume ratio as to completely dissolve the aforementioned mixture. The reaction occurs with a yield of not less than 95% when nimesulide and tetramethylammonium hydroxide pentahydrate are mixed at a molar ratio of 1:1, which is favourable. The reaction mixture is stirred after the addition of the solvent or solvent mixture until the substrates are completely dissolved at room temperature, e.g. about 1 minute. The resulting solution is then left at room temperature to slowly evaporate in a place out of sunlight for several days until the substance crystallises.

The method for producing crystals of tetraethylammonium salt of nimesulide is that nimesulide and an aqueous solution of tetraethylammonium hydroxide are mixed together at any weight/mass % with respect to the molar ratio depending on the intended number of grams of product and the yield of the reaction. The reaction occurs with a yield of not less than 95% only when nimesulide and tetraethylammonium hydroxide are mixed in a molar ratio of 1:1, which is an advantageous feature of the invention. The reaction mixture is stirred for about 1 minute until the substrates are completely dissolved at room temperature, e.g. about 1 minute. After mixing the components, the resulting solution is left at room temperature to slowly evaporate water in a place without sunlight for several days until the substance crystallises.

Advantages resulting from the invention:
The substances according to the invention have good water solubility: tetramethylammonium salt of nimesulide at least 1.5 mg/mL, tetraethylammonium salt of nimesulide at least 1.4 mg/mL, which is at least 100 times higher than the water solubility of nimesulide of 0.01 mg/mL, which makes them have higher bioavailability than pure nimesulide.

The object of the invention is illustrated in more detail in the examples of its manufacture and the drawings, where: Fig. 1 shows the asymmetric part of the elementary cell of a tetramethylammonium nimesulide salt with the numbering of atoms and hydrogen bonds indicated by the dashed line, Fig. 2 shows the packing of ions in the crystal lattice of a tetramethylammonium nimesulide salt showing the formation of layers through hydrogen bonds (indicated by the dashed line), Fig. 3 shows the TG/DTG profile of the crystals of a tetramethylammonium nimesulide salt. Fig. 4 shows the melting temperature diagram of the tetramethylammonium nimesulide salt crystals made on a BUCHI cryometer. Fig. 5 shows the asymmetric part of the elementary cell of tetraethylammonium nimesulide salt with the numbering of atoms and hydrogen bonds indicated by the dashed line, Fig. 6 shows the packing of ions in the crystal lattice of tetraethylammonium nimesulide salt showing the formation of layers through hydrogen bonds (indicated by the dotted line), Fig. 7 shows the TG/DTG profile of crystals of tetraethylammonium nimesulide salt. Fig. 8 shows a melting temperature plot of tetraethylammonium nimesulide salt crystals made on a BUCHI cryometer.

### Example 1

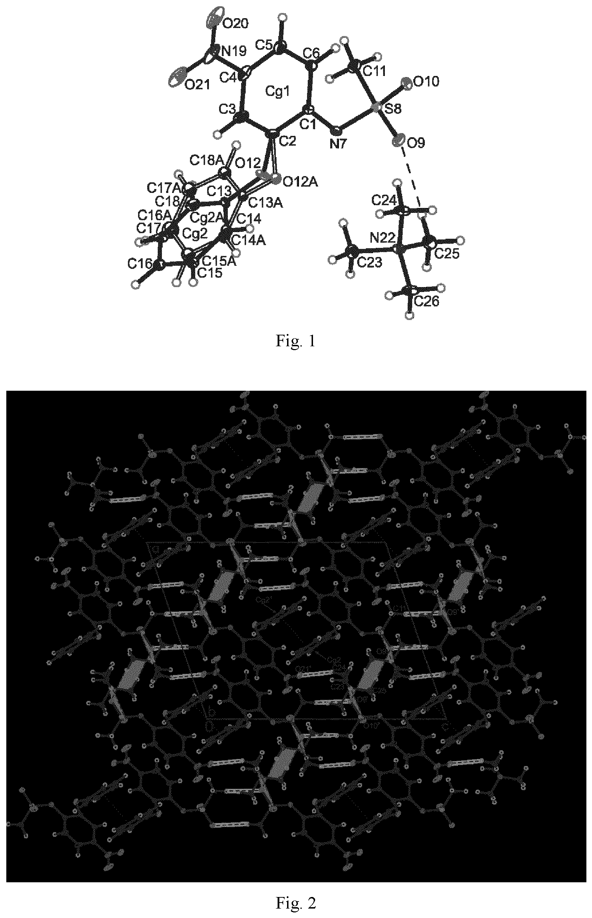

0.05 g of nimesulide (0.162 mmol) and 0.026 g of tetramethylammonium hydroxide pentahydrate (0.162 mmol) were weighed, added to a mixture of solvents: 10 cm³ ethanol and 5 cm³ methanol, and stirred for 1 minute at room temperature The beaker with the solution was left at room temperature to evaporate in a place out of sunlight. After 14 days, yellow crystals were collected at the bottom of the beaker. The product obtained was tetramethylammonium nimesulide salt crystals, as confirmed by X-ray structural analysis XRD, TG/DTG profile and melting point graph shown in Table 1 and Fig. 1-4. X-ray structural analysis was carried out at 20°C on a Gemini R Ultra four-wheel diffractometer with Ruby CCD detector, from Oxford Diffraction using radiation at λ_{Mo} =0.71073 Å. Data registration, reduction and analysis were performed using CRYSALIS-CCD and CRYSALIS-RED software. The structure was resolved by direct methods using SHELXS-2013 and refined using SHELXL-17, as shown in Table 1.

**Table 1. Selected crystallographic data for the tetramethylammonium salt of nimesulide**

| | |
|---|---|
| Empirical formula | C₁₇ H₂₃ N₃ O₅ S |
| Molar mass [g/mol] | 381,44 |
| Measurement temperature [K] | 293(2) |
| Wavelength of radiation used [Å] | 0,71073 |
| Crystallographic arrangement | single-sided |
| Spatial group | P2₁ /n |
| Elementary cell parameters | a = 13,7425(18) [Å] α = 90 [°] |
| | b = 8.3439(9) [Å] β = 108,752(14) [°] |
| | c = 17.529(2) [Å] γ = 90 [°] |
| Volume of the elementary cell [Å³] | 1903,3(4) |
| Number of molecules in an elementary cell | 4 |
| Theoretical crystal density [mg/m³ ] | 1,331 |
| Number of electrons in an elementary cell | 808 |
| Radiation range of angle θ [°] | 3,30-25,00 |
| Number of reflexes/nodes/parameters to be refined | 3328 / 66 / 269 |
| Divergence index for reflections | R1 = 0,0785 |
| I>2σ(I) | wR2 = 0,1763 |
| Divergence index for all reflections | R1 = 0,1275 |
| | wR2 = 0.2084 |
| Finishing quality index [F² ] | 1,039 |

It can be seen from Table 1 that the tetramethylammonium salt of nimesulide crystallises in the P2 space group₁ /n of the single-stranded system. The crystal of the compound under study contains one nimesulide anion and one tetramethylammonium cation in the asymmetric part, as shown in Fig. 1. Analysis of packing in the crystal lattice proves the formation of layers in the structure through the presence of hydrogen bonds and π-π interactions, as shown in Fig. 2.

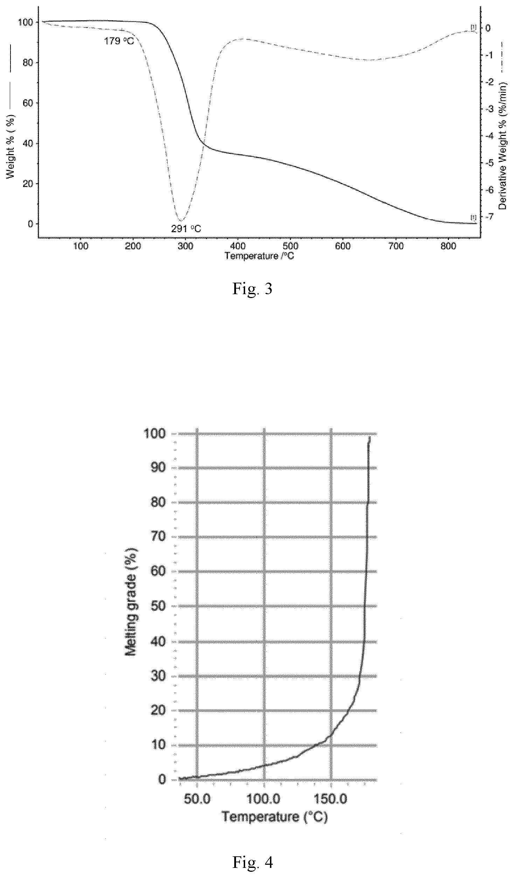

Thermogravimetric analysis was performed with a NETZSCH TG 209 apparatus in an argon atmosphere. The samples were tested in the range 24 - 1000 °C with a heating rate of 10 °C min on an Al₂O₃ crucible, as shown in Fig. 3. The melting temperature of the crystals was measured using a BUCHI M-565 cryometer. Measured samples of the crystals were tested in capillaries with a step of 1 °C/min in the range 50 - 400 °C, as shown in Fig. 4. 4. The TG/DTG thermogram visible in Fig. 3 shows two decomposition temperatures for the tetramethylammonium salt of nimesulide 179.0 and 291.0 °C - the former is also observed in the melting temperature diagram, as shown in Fig. 4.

Tests conducted to determine the solubility of the tetramethylammonium salt of nimesulide show that the substance according to the invention has a good solubility in water of at least 1.5 mg/mL, which is at least 100 times higher than the solubility of nimesulide in water of 0.01 mg/mL.

### Example 2

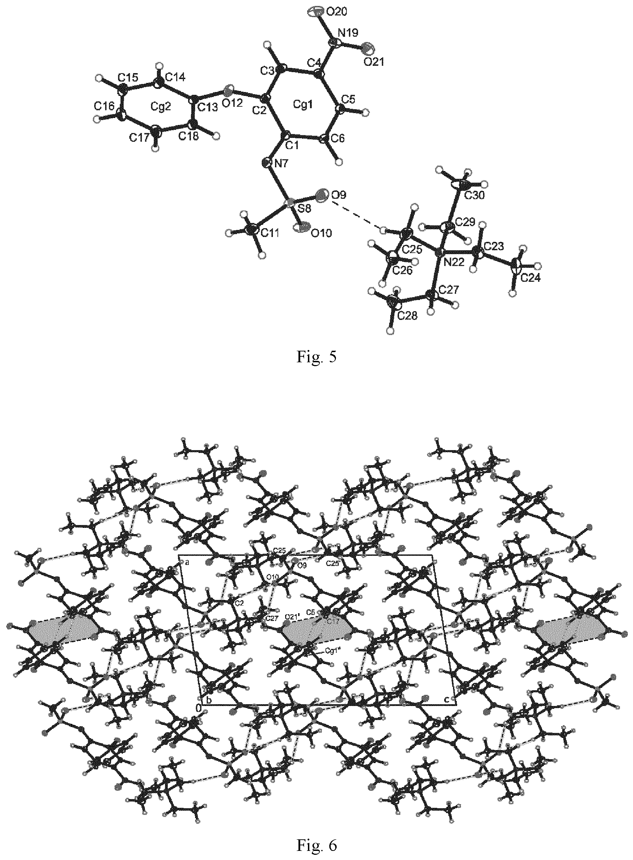

0.05 g of nimesulide (0.162 mmol) was weighed and 0.12 ml of an aqueous solution of tetraethylammonium hydroxide (20% by weight in H₂ O, d=1.01 g/cm³ at 20°C) containing 0.162 mmol of tetraethylammonium hydroxide was measured and stirred for 1 min at room temperature. The beaker with the solution was left at room temperature until the water evaporated in a place out of sunlight. After 15 days, yellow crystals were collected at the bottom of the beaker. The product obtained was tetraethylammonium nimesulide salt crystals, as confirmed by X-ray structural analysis XRD, TG/DTG profile and melting point plot shown in Table 2 and Figs. 5-8. X-ray structural analysis was carried out at 20°C on a Gemini R Ultra four-wheel diffractometer with Ruby CCD detector, from Oxford Diffraction using radiation at λ_{Mo} =0.71073 Å. Data registration, reduction and analysis were performed using CRYSALIS-CCD and CRYSALIS-RED software. The structure was resolved by direct methods using SHELXS-2013 and refined using SHELXL-17, as shown in Table 2.

**Table 2. Selected crystallographic data for the tetraethylammonium salt of nimesulide**

| | |
|---|---|
| Empirical formula | C₂₁ H₃₁ N₃ O₅ S |
| Molar mass [g/mol] | 437,55 |
| Measurement temperature [K] | 293(2) |
| Wavelength of radiation used [Å] | 0,71073 |
| Crystallographic arrangement | single-sided |
| Spatial group | P2₁ /n |
| Elementary cell parameters | a = 12,0108(7) [Å] α = 90 [°] |
| | b=11.5166(7) [Å]. β = 97,564(5) [°] |
| | c = 16.7784(9) [Å] γ = 90 [°] |
| Volume of the elementary cell [Å³] | 2300,6(2) |
| Number of molecules in an elementary cell | 4 |
| Theoretical crystal density [mg/m³ ] | 1,263 |
| Linear absorption coefficient, µ [mm⁻¹ ] | 0,176 |
| Radiation range of angle θ [°] | 3,63 - 25,00 |
| The number of finetuned reflections/nodes/parameters | 4043 / 0 / 276 |
| Divergence index for reflections | R1 = 0,0597 |
| I>2σ(I) | wR2 = 0.1605 |
| Divergence index for all reflections | R1 = 0,0767 |
| | wR2 = 0,1729 |
| Precision quality index [F² ] | 1,087 |

It can be seen from Table 2 that the tetraethylammonium salt of nimesulide crystallises in the P2 space group₁ /n of the single-stranded system. The crystal of the compound under study contains one nimesulide anion and one tetraethylammonium cation in the asymmetric part, as shown in Fig. 5. Analysis of packing in the crystal lattice shows the formation of layers in the structure through the presence of hydrogen bonds and C-H π interactions, as shown in Fig. 6.

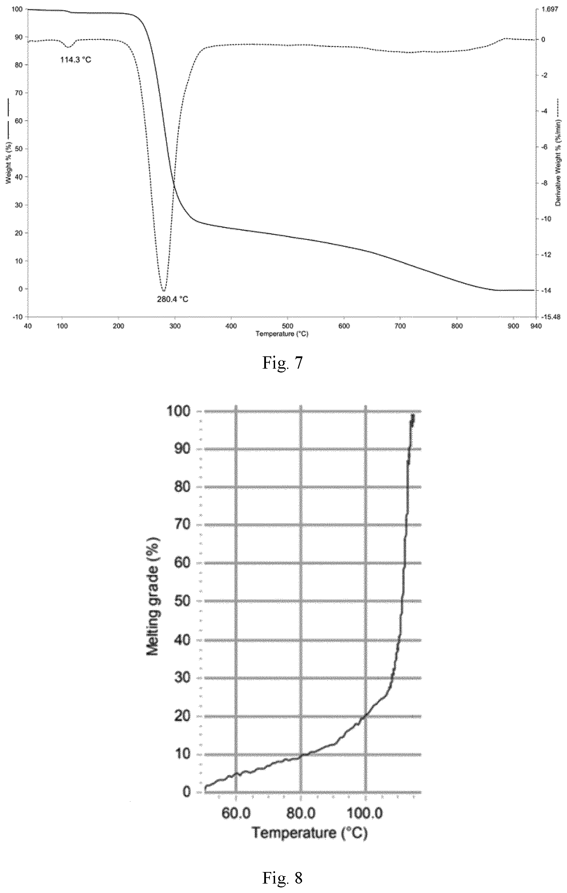

Thermogravimetric analysis was performed with a NETZSCH TG 209 apparatus in an argon atmosphere. Tests for the samples were performed in the range 24 - 1000 °C with a heating rate of 10 °C min on an Al₂O₃ crucible , as shown in Fig. 7. The melting temperature of the crystals was measured using a BUCHI M-565 cryometer. Measured samples of the crystals were examined in capillaries with a step of 1 °C/min in the range 50 - 400 °C, as shown in Fig. 8. 8. The TG/DTG thermogram shown in Fig. 7 describes two decomposition temperatures for the tetraethylammonium salt of nimesulide 114.3 and 280.4 °C - the former is also observed in the melting temperature diagram, as shown in Fig. 8.

Tests conducted to determine the solubility of the tetraethylammonium salt of nimesulide show that the substance according to the invention has a good solubility in water of at least 1.4 mg/mL, which is at least 100 times higher than the solubility of nimesulide in water of 0.01 mg/mL.

## Claims

1. Tetramethylammonium salt of nimesulide with formula 1: 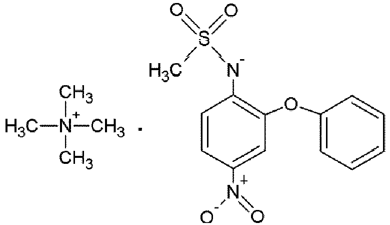 where nimesulide occurs in an ionised form.

2. The tetramethylammonium salt of nimesulide according to claim 1, which occurs as a solid crystalline, including its polymorphs and solvates.

3. Tetraethylammonium salt of nimesulide with formula 2: 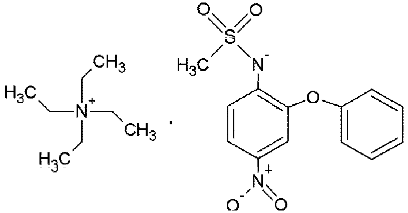 where nimesulide occurs in an ionised form.

4. The tetraethylammonium salt of nimesulide according to claim 3, which occurs as a solid crystalline, including its polymorphs and solvates..

5. Method for obtaining the tetramethylammonium salt of nimesulide of formula 1 according to claim 1,
**characterised in that** it comprises preparing a mixture of nimesulide and a compound constituting a source of tetramethylammonium ions, preferably in an equimolar ratio, and dissolving them in a solvent or mixtures of solvents, and leaving the solution thus obtained until the solvent is completely evaporated and the compound crystallised.

6. Method according to claim 5, **characterised in that** tetramethylammonium hydroxide pentahydrate is used.

7. Method for the preparation of the tetraethylammonium salt of nimesulide of formula 2 according to claim 3,
**characterised in that** it comprises preparing a mixture of nimesulide and a compound as a source of tetraethylammonium ions, preferably in an equimolar ratio, and dissolving them in a solvent or mixtures of solvents, and leaving the solution thus obtained until the solvent is completely evaporated and the compound crystallises.

8. Method according to claim 7, **characterised in that** an aqueous solution of tetraethylammonium hydroxide is used.

## Patentansprüche

1. Tetramethylammoniumsalz von Nimesulid mit der Formel 1: 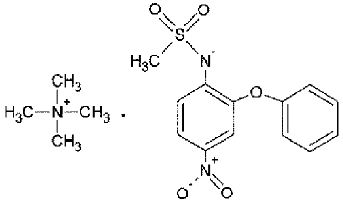 wobei Nimesulid in ionisierter Form vorkommt.

2. Tetramethylammoniumsalz von Nimesulid gemäß Anspruch1, das als festes Kristallin vorkommt, inklusive Polymorphe und Solvate.

3. Tetramethylammoniumsalz von Nimesulid mit der Formel 2: 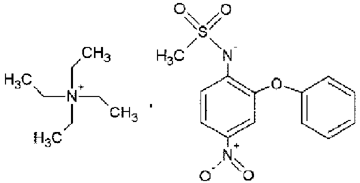 wobei Nimesulid in ionisierter Form vorkommt.

4. Tetramethylammoniumsalz von Nimesulid, gemäß Anspruch 3, das als festes Kristallin vorkommt, inklusive Polymorphe und Solvate.

5. Methode zur Erstellung von Tetramethylammoniumsalz von Nimesulid nach Formel 1, gemäß Anspruch 1, **gekennzeichnet dadurch, dass** es die Herstellung einer Mischung aus Nimesulid und einer Verbindung für Tetramethylammoniumione darstellt, vorzugsweise in einem äquimolaren Verhältnis, und das Auflösen dieser Mischungen in einem Lösungsmittel oder Lösungsmittelgemischen umfasst, und das Stehenlassen der so erhaltenen Lösung, bis das Lösungsmittel völlig verdampft und die Verbindung kristallisiert ist.

6. Methode gemäß Anspruch 5, **gekennzeichnet dadurch, dass** Tetramethylammoniumhydroxid-Pentahydrat verwendet wird.

7. Methode zur Erstellung von Tetramethylammoniumsalz von Nimesulid nach Formel 2, gemäß Anspruch 3, **gekennzeichnet dadurch, dass** es die Herstellung einer Mischung aus Nimesulid und einer Verbindung für Tetramethylammoniumione umfasst, vorzugsweise in einem äquimolaren Verhältnis, und das Auflösen dieser Mischungen in einem Lösungsmittel oder Lösungsmittelgemischen umfasst, und das Stehenlassen der so erhaltenen Lösung, bis das Lösungsmittel völlig verdampft und die Verbindung kristallisiert ist.

8. Methode gemäß Anspruch 7, **gekennzeichnet dadurch, dass** eine wasserhaltige Lösung verwendet wird.

## Revendications

1. Sel de nimésulide de tétraméthylammonium ayant la formule 1 : 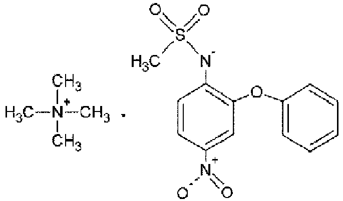 où le nimésulide se présente sous forme ionisée.

2. Le sel de nimésulide de tétraméthylammonium selon la revendication 1, se présentant sous forme cristalline solide, incluant ses polymorphes et solvats.

3. Sel de nimésulide de tétraéthylammonium ayant la formule 2 : 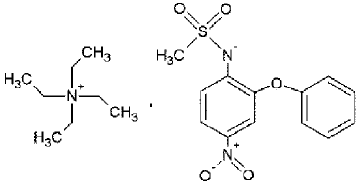 où le nimésulide est présent sous forme ionisée.

4. Le sel de tétraéthylammonium du nimésulide, selon la revendication 3, se présentant sous forme cristalline solide, incluant ses polymorphes et solvats.

5. Méthode d'obtention du sel de tétraméthylammonium du nimésulide de formule 1, selon la revendication 1, **caractérisée en ce qu'**elle consiste à préparer un mélange de nimésulide et d'un composé constituant une source d'ions tétraméthylammonium, de préférence dans un rapport équimolaire, puis à les dissoudre dans un solvant ou un mélange de solvants, et à laisser la solution obtenue jusqu'à ce que le solvant soit complètement évaporé et que le composé cristallise.

6. Méthode selon la revendication 5, **caractérisée en ce que** l'hydroxyde de tétraméthylammonium pentahydraté est utilisé.

7. Méthode de préparation du sel de tétraéthylammonium du nimésulide de formule 2, selon la revendication 3, **caractérisée en ce qu'**elle consiste à préparer un mélange de nimésulide et d'un composé servant de source d'ions tétraéthylammonium, de préférence dans un rapport équimolaire, puis à les dissoudre dans un solvant ou un mélange de solvants, et à laisser la solution obtenue jusqu'à ce que le solvant soit complètement évaporé et que le composé cristallise.

8. Méthode selon la revendication 7, **caractérisée en ce qu'**une solution aqueuse d'hydroxyde de tétraéthylammonium est utilisée.
